# EUROPÄISCHE PATENTSCHRIFT

(11) **EP 1 263 712 B1**
(45) Veröffentlichungstag und Bekanntmachung des Hinweises auf die Patenterteilung: **14.12.2005**
(21) Anmeldenummer: 01273996.7
(22) Anmeldetag: 18.10.2001
(51) Int. Cl.: C07C 237/20, C07D 295/18, A61K 7/13

(54) **3-(2,5-DIAMINOPHENYL)-ACRYLAMID-DERIVATE UND DIESE VERBINDUNGEN ENTHALTENDE FÄRBEMITTEL**
3-(2,5-DIAMINOPHENYL)-ACRYLAMIDE DERIVATIVES AND COLOURING AGENTS CONTAINING SAID COMPOUNDS
DERIVES DE 3-(2,5-DIAMINOPHENYL)-ACRYLAMIDE ET COLORANTS LES CONTENANT

(30) Priorität: 17.03.2001 DE 10113027
(43) Veröffentlichungstag der Anmeldung: 11.12.2002
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(86) Internationale Anmeldenummer: PCT/EP2001/012054
(87) Internationale Veröffentlichungsnummer: WO 2002/074731

(56) Entgegenhaltungen:
- EP-A- 0 007 537
- EP-A- 0 797 980
- WO-A-99/59527

## Beschreibung

Die Erfindung betrifft neue 3-(2,5-Diaminophenyl)-acrylamid-Derivate sowie diese Verbindungen enthaltende Mittel zum Färben von Keratinfasern.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol 1,4-Diaminobenzol und 4,5-Diamino-1-(2-hydroxyethyl)-pyrazol eingesetzt, während als Kupplersubstanzen beispielsweise Resorcin, 2-Methylresorcin, 1-Naphthol, 3-Aminophenol, m-Phenylendiamin, 2-Amino-4-(2'-hydroxyethyl)amino-anisol, 1,3-Diamino-4-(2'-hydroxyethoxy)benzol und 2,4-Diamino-5-fluor-toluol zu nennen

Zur Abdeckung des wichtigen Blondbereichs wurden Oxidationshaarfärbemitteln bisher insbesondere direktziehende aromatische Nitrofarbstoffe zugesetzt.

Die Verwendung von substituierten p-Phenylendiaminen als Farbkomponente in Oxidationshaarfärbemitteln ist aus der Literatur, beispielsweise der DE-OS 198 22 041, EP-OS 0 634 163,

EP-OS 0 819 424, DE-OS 39 17 304 und EP-OS 0 007 537, bekannt. Diese Verbindungen erfüllen jedoch die an Farbstoffe für Oxidationsfärbemittel für den Blondbereich gestellten Anforderungen nicht in jeder Hinsicht. Es bestand daher weiterhin ein Bedarf nach geeigneten neuen Farbstoffen.

Es wurde nunmehr gefunden, dass bestimmte p-Phenylendiaminderivate zusammen mit üblichen Kupplerverbindungen eine intensiv blonde Färbung von Fasern, insbesondere Keratinfasern wie zum Beispiel Haaren, ermöglichen. So werden bei Verwendung dieser p-Phenylendiaminderivate in oxidierendem Medium farbstarke Farbnuancen erhalten, die außerordentlich lichtecht und waschecht sind.

Gegenstand der vorliegende Erfindung sind daher 3-(2,5-Diaminophenyl)-acrylamid-Derivate der allgemeinen Formel (I) oder deren physiologisch verträglichen, wasserlöslichen Salze, 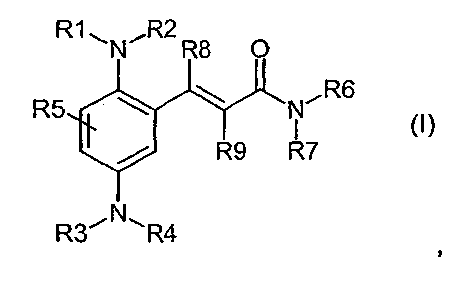 worin
**R1, R2, R3 und R4** gleich oder verschieden sein können und unabhängig voneinander Wasserstoff, eine C₁-C₆-Alkylgruppe, eine C₂-C₄-Hydroxyalkylgruppe oder eine C₃-C₄-Dihydroxyalkylgruppe darstellen oder **R1** und **R2** beziehungsweise **R3** und **R4** einen viergliedrigen bis achtgliedrigen aliphatischen Ring bilden, wobei mindestens 2 der Reste **R1** bis **R4** Wasserstoff darstellen;
**R5** gleich Wasserstoff, einem Halogenatom (F, Cl, Br, J), einer C₁-C₄-Alkylgruppe, einer C₁-C₄ -Hydroxyalkylgruppe oder einer C₁-C₄-Alkoxygruppe ist;
**R6** und **R7** gleich oder verschieden sein können und unabhängig voneinander gleich Wasserstoff, einer C₁-C₂-Alkoxygruppe, einer C₁-C₆-Alkylgruppe, einer ungesättigten C₃-C₆-Alkylgruppe, einer C₂-C₄-Hydroxyalkylgruppe, einer C₃-C₄-Dihydroxyalkylgruppe, einer C₂-C₄-Aminoalkylgruppe, einer C₂-C₄-Dimethylaminoalkylgruppe, einer C₂-C₄-Acetylaminoalkylgruppe einer C₂-C₄-Methoxyalkylgruppe, einer C₂-C₄-Ethoxyalkylgruppe, einer C₁-C₄-Cyanalkylgruppe, einer C₁-C₄-Carboxyalkylgruppe, einer C₂-C₄-Aminocarbonylalkylgruppe, einer Pyridylmethylgruppe, einer Furfurylgruppe, einer hydrierten Furfurylgruppe, einer substituierten Pyridylgruppe, einem Rest der Formel (II) 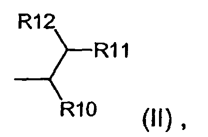 einem Rest der Formel (III) 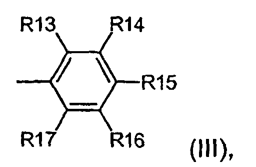 oder einem Rest der Formel (IV) 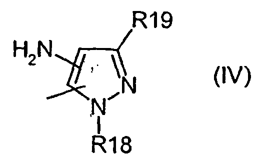 sind, oder **R6** und **R7** einen Ring der Formel 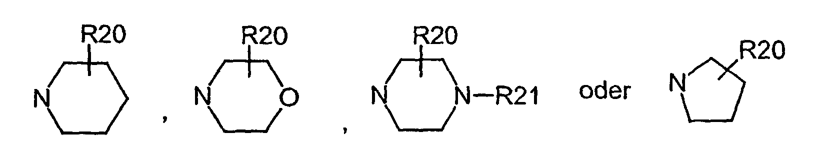 bilden;
**R8, R9** und **R21** gleich oder verschieden sein können und unabhängig voneinander gleich Wasserstoff, oder einer C₁-C₆ Alkylgruppe sind;
**R10** gleich Wasserstoff, einer Carboxygruppe, oder einer Aminocarbonylgruppe ist;
**R11** und **R12** gleich oder verschieden sein können und unabhängig voneinander gleich Wasserstoff, einer Hydroxygruppe, einer Aminocarbonylgruppe, einer Methylthiomethylgruppe, einem mit einer Phenylgruppe oder Hydroxygruppe substituierten Phenylrest oder einem Rest der Formel 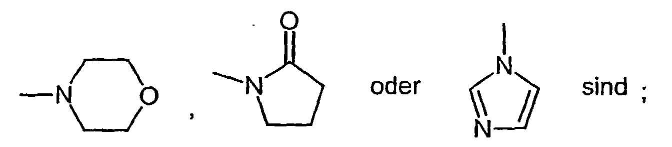 **R13, R14, R15, R16** und **R17** gleich oder verschieden sein können und unabhängig voneinander gleich Wasserstoff, einem Halogenatom (F, Cl, Br, J), einer Cyanogruppe, einer Hydroxygruppe, einer-C₁-C₄-Alkoxygruppe, einer C₁-C₄-Hydroxyalkoxygruppe, einer C₁-C₆-Alkylgruppe, einer C₁-C₄-Alkylthioethergruppe, einer Mercaptogruppe, einer Nitrogruppe, einer Aminogruppe, einer Alkylaminogruppe, einer Hydroxyalkylaminogruppe, einer Dialkylaminogruppe, einer Di(hydroxyalkyl)aminogruppe, einer (Dihydroxyalkyl)aminogruppe, einer (Hydroxyalkyl)alkylaminogruppe, einer Trifluormethangruppe, einer -C(O)H-Gruppe, einer -C(O)CH₃ Gruppe, einer -C(O)CF₃-Gruppe, einre -Si(CH₃)₃-Gruppe, einer C₁-C₄-Hydroxyalkylgruppe, einer C₂-C₄ Dihydroxyalkylgruppe sind, oder zwei nebeneinanderliegende Reste **R13** bis **R17** gemeinsam eine -O-CH2-O-Brücke bilden;
**R18** gleich einer C₁-C₄-Alkylgruppe einer Benzylgruppe oder einer C₁-C₄-Hydroxyalkylgruppe ist;
**R19** gleich Wasserstoff oder einer C₁-C₆ Alkylgruppe ist;
**R20** gleich Wasserstoff, einer Hydroxygruppe, einer Carboxygruppe, einer Aminocarbonylgruppe oder einer Hydroxymethylgruppe ist.

Als Verbindungen der Formel (I) können beispielweise genannt werden:
3-(2,5-Diaminophenyl)-N-methyl-acrylamid, 3-(2,5-Diaminophenyl)-N-propyl-acrylamid, 3-(2,5-Diaminophenyl)-1-morpholin-4-yl-propenon, 3-(2,5-Diaminophenyl)-N-(1-hydroxymethylpropyl)-acrylamid, 3-(2,5-Diaminophenyl)-N-furan-2-ylmethyl-acrylamid, 3-(2,5-Diaminophenyl)-N-methoxy-N-methyl-acrylamid, 3-(2,5-Diaminophenyl)-N-(2-hydroxy-1-methyl-ethyl)-acrylamid, N-(2-Aminoethyl)-3-(2,5-diaminophenyl)-acrylamid, 3-(2,5-Diaminophenyl)-N-(tetrahydrofuran-2-yl-methyl)-acrylamid, N-Cyclopropyl-3-(2,5-diamino-phenyl)-acrylamid, 3-(2,5-Diaminophenyl)-N-isopropyl-acrylamid,3-(2,5-Diaminophenyl)-N-(2-methoxyethyl)-acrylamid, 3-(2,5-Diaminophenyl)-1-(4-hydroxy-piperidin-1-yl)-propenon, N-(2-Acetylaminoethyl)-3-(2,5-diaminophenyl)-acrylamid, 3-(2,5-Diamino-phenyl)-N-(2-morpholin-4-yl-ethyl)-acrylamid, 3-(2,5-Diaminophenyl)N-[3-(2-oxo-pyrrolidin-1-yl)-propyl)-acrylamid, 2-[3-(2,5-Diaminophenyl)-acryloylamino]-3-methyl-buttersäure, [3-(2,5-Diaminophenyl)-acryloylamino]-essigsäure, N-Allyl-3-(2,5-diaminophenyl)-acrylamid, 3-(2,5-Diaminophenyl)-N-[2-(5-nitro-pyridin-2-ylamino)-ethyl]-acrylamid, 3-(2,5-Diaminophenyl)-N-(3-imidazol-1-yl-propyl)-acrylamid, 3-(2,5-Diaminophenyl)-N-[2-(3H-imidazol-4-yl)-ethyl]-acrylamid, N-(4-Aminophenyl)-3-(2,5-diaminophenyl)-acrylamid, 3-(2,5-Diaminophenyl)-N-[2-(4-sulfamoyl-phenyl)-ethyl]-acrylamid, N-[5-Chlor-4-(2-hydroxyethylamino)-2-nitro-phenyl]-3-(2,5-diamino-phenyl)-acrylamid, 3-(2,5-Diaminophenyl)-1-pyrrolidin-1-yl-propenon, 3-(2,5-Diaminophenyl)-N-pyridin-2-yl-acrylamid, N-[3-(2,5-Diaminophenyl)-prop-2-enoyl]-L-glutamin-säure-Trifluoracetat, N2-[3-(2,5-diaminophenyl)-prop-2-enoyl]-glutamin-Trifluoracetat, N-[4-Amino-2-(2-hydroxyethyl)-2H-pyrazol-3-yl]-3-(2,5-diaminophenyl)-acrylamid, N-(4-Amino-2(3)-methyl-phenyl)-3-(2,5-diaminophenyl)-acrylamid, N-[4-Amino-2(3)-(2-hydroxy-ethyl)-phenyl]-3-(2,5-diaminophenyl)-acrylamid, N-{4-[Bis-(2-hydroxy-ethyl)-amino]-phenyl}-3-(2,5-diaminophenyl)-acrylamid, 3-(2,5-Diamino-phenyl)-N-(4-hydroxyphenyl)-acrylamid, 3-(2,5-Diaminophenyl)-N-(3-hydroxy-4-methylphenyl)-acrylamid, N-(3-Aminophenyl)-3-(2,5-diamino-phenyl)-acrylamid, 3-(2,5-Diaminophenyl)-N-(3-hydroxy-5-hydroxymethyl-2-methyl-pyridin-4-ylmethyl)-acrylamid, 3-(2,5-Diaminophenyl)-N-(2-hydroxy-5-nitro-phenyl)-acrylamid, N-(3-Chlor-2-hydroxy-5-nitro-phenyl)-3-(2,5-diaminophenyl)-acrylamid, 3-(2,5-Diaminophenyl)-1-(2-hydroxy-methyl-pyrrolidin-1-yl)-propenon, 3-(2,5-Diaminophenyl)-1-(3-hydroxy-pyrrolidin-1-yl)-propenon, 1-[3-(2,5-Diaminophenyl)-acryloyl]-pyrrolidin-2-carbonsäureamid, 3-(2,5-Diaminophenyl)-1-(3-hydroxy-piperidin-1-yl)-propenon, 3-(2,5-Diaminophenyl)-N-(2-hydroxy-1-hydroxymethylethyl)-acrylamid, N-(1-Carbamoyl-2-hydroxyethyl)-3-(2,5-diaminophenyl)-acrylamid, N-[3-(2,5-Diaminophenyl)-prop-2-enoyl]-L-asparaginsäure. N2-[3-(2,5-Diaminophenyl)-prop-2-enoyl]-L-asparagin. N-[3-(2,5-Diaminophenyl)-prop-2-enoyl]-L-leucin , N-[5-Amino-2(4)-(2-hydroxyethoxy)-phenyl]-3-(2,5-diaminophenyl)-acrylamid, 3-(2,5-Diaminophenyl)-N-ethyl-acrylamid, N-Benzo[1,3]dioxol-5-yl-3-(2,5-diamino-phenyl)-acrylamid, 2-(3-(2,5-Diaminophenyl)-acryloylamino]-4-methyl-pentansäure, 3-(2,5-Diaminophenyl)-1-(6,7-dihydroxy-1-methyl-3,4-dihydro-1H-isochinolin-2-yl)-propenon, N-(4-Aminophenyl)-3-(N²,N²-bis-methyl-2,5-diaminophenyl)-acrylamid, N-(4-Aminophenyl)-3-(N⁵,N⁵-bis-methyl-2,5-diaminophenyl)-acrylamid, N-(4-Aminophenyl)-3-(N²,N²-bis-(2-hydroxyethyl)-2,5-diaminophenyl)-acrylamid, N-(4-Aminophenyl)-3-(N⁵,N⁵-bis-(2-hydroxyethyl)-2,5-diaminophenyl)-acrylamid; N-(4-Aminophenyl)-3-(N²-(2,3-dihydroxypropyl)-2,5-diaminophenyl)-acrylamid, N-(4-Aminophenyl)-3-(N⁵-(2,3-dihydroxypropyl)-2,5-diaminophenyl)-acrylamid, N-(4-Aminophenyl)-3-(2-(pyrrolidin-1-yl)-5-aminophenyl)-acrylamid, N-(4-Aminophenyl)-3-(5-(pyrrolidin-1-yl)-2-aminophenyl)-acrylamid, 3-(N²,N²-bis-methyl-2,5-diaminophenyl)-N-ethyl-acrylamid, 3-(N⁵,N⁵-bis-methyl-2,5-diaminophenyl)-N-ethyl-acrylamid, 3-(N²,N²-bis-(2-hydroxyethyl)-2,5-diaminophenyl)-N-ethyl-acrylamid, 3-(N⁵,N⁵-bis-(2-hydroxyethyl)-2,5-diaminophenyl)-N-ethyl-acrylamid; 3-(N²-(2,3-dihydroxypropyl)-2,5-diaminophenyl)-N-ethyl-acrylamid, 3-(N⁵-(2,3-dihydroxypropyl)-2,5-diaminophenyl)-N-ethyl-acrylamid, 3-(2-(pyrrolidin-1-yl)-5-aminophenyl)-N-ethyl-acrylamid, 3-(5-(pyrrolidin-1-yl)-2-aminophenyl)-N-ethyl-acrylamid, 3-(N²,N²-bis-methyl-2,5-diaminophenyl)-N-propyl-acrylamid, 3-(N⁵,N⁵-bis-methyl-2,5-diaminophenyl)-N-propyl-acrylamid, 3-(N²,N²-bis-(2-hydroxyethyl)-2,5-diaminophenyl)-N-propyl-acrylamid, 3-(N⁵,N⁵-bis-(2-hydroxyethyl)-2,5-diaminophenyl)-N-propyl-acrylamid; 3-(N²-(2,3-dihydroxypropyl)-2,5-diaminophenyl)-N-propyl-acrylamid, 3-(N⁵-(2,3-dihydroxypropyl)-2,5-diaminophenyl)-N-propyl-acrylamid, 3-(2-(pyrrolidin-1-yl)-5-aminophenyl)-N-propyl-acrylamid, 3-(5-(pyrrolidin-1-yl)-2-amino-phenyl)-N-propyl-acrylamid, 3-(N2,N2-bis-methyl-2,5-diaminophenyl)-N-(2-hydroxy-1-methylethyl)-acrylamid, 3-(N⁵,N⁵-bis-methyl-2,5-diaminophenyl)-N-(2-hydroxy-1-methylethyl)-acrylamid, 3-(N²,N²-bis-(2-hydroxyethyl)-2,5-diaminophenyl)-N-(2-hydroxy-1-methylethyl)-acrylamid, 3-(N⁵,N⁵-bis-(2-hydroxyethyl)-2,5-diaminophenyl)-N-(2-hydroxy-1-methylethyl)-acrylamid; 3-(N2-(2,3-dihydroxypropyl)-2,5-diaminophenyl)-N-(2-hydroxy-1-methylethyl)-acrylamid, 3-(N5-(2,3-dihydroxypropyl)-2,5-diaminophenyl)-N-(2-hydroxy-1-methylethyl)-acrylamid, 3-(2-(pyrrolidin-1-yl)-5-aminophenyl)-N-(2-hydroxy-1-methylethyl)-acrylamid, 3-(5-(pyrrolidin-1-yl)-2-aminophenyl)-N-(2-hydroxy-1-methylethyl)-acrylamid oder deren physiologisch verträglichen Salze.

Bevorzugt sind Verbindungen der Formel (I), bei denen (i) eine oder mehrere der Restgruppen **R5, R8** und **R9** gleich Wasserstoff sind und/oder (ii) **R1, R2, R3** und **R4** gleich Wasserstoff sind und/oder (iii) **R6** gleich einer Methylgruppe, einer Methoxygruppe oder einer C₂-C₄-Hydroxyalkylgruppe und **R7** gleich einer C₂-C₄-Hydroxyalkylgruppe ist und/oder (iv) **R6** gleich Wasserstoff und **R7** gleich einer C₁-C₆-Alkylgruppe, einer ungesättigten C₃-C₆ -Alkylgruppe, einer C₂-C₄-Hydroxyalkylgruppe, einem Furfurylrest, einem substituierten Phenylrest oder einem subsituierten Pyrazoylrest ist, und/oder (v) **R6** und **R7** einen aliphatischen Ring der Formel 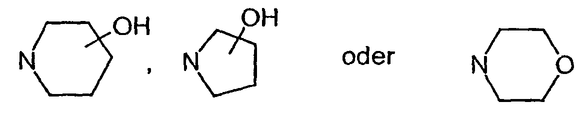 bilden.

Insbesondere sind die folgenden Verbindungen zu nennen:
3-(2,5-Diaminophenyl)-N-propyl-acrylamid, 3-(2,5-Diaminophenyl)-N-furan-2-ylmethyl-acrylamid, 3-(2,5-Diaminophenyl)-N-methoxy-N-methyl-acrylamid, 3-(2,5-Diaminophenyl)-N-(2-hydroxy-1-methylethyl)-acrylamid, N-Cyclopropyl-3-(2,5-diaminophenyl)-acrylamid, 3-(2,5-Diaminophenyl)-N-isopropyl-acrylamid, N-(4-Aminophenyl)-3-(2,5-diaminophenyl)-acrylamid, 3-(2,5-Diaminophenyl)-1-pyrrolidin-1-yl-propenon, N-[4-Amino-2-(2-hydroxyethyl)-2H-pyrazol-3-yl]-3-(2,5-diaminophenyl)-acrylamid, N-(4-Amino-2(3)-methylphenyl)-3-(2,5-diaminophenyl)-acrylamid, N-{4-[Bis-(2-hydroxyethyl)amino]-phenyl}-3-(2,5-diaminophenyl)-acrylamid, 3-(2,5-Diaminophenyl)-N-(4-hydroxyphenyl)-acrylamid, 3-(2,5-Diaminophenyl)-1-(3-hydroxy-pyrrolidin-1-yl)-propenon, N-[5-Amino-2(4)-(2-hydroxyethoxy)-phenyl]-3-(2,5-diaminophenyl)-acrylamid, 3-(2,5-Diaminophenyl)-N-ethyl-acrylamid.

Die 3-(2,5-Diaminophenyl)-acrylamid-Derivate der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen 3-(2,5-Diaminophenyl)-acrylamid-Derivate der Formel (I) kann unter Verwendung von bekannten Syntheseverfahren erfolgen. Die Synthese der erfindungsgemäßen Verbindungen kann beispielsweise wie folgt durchgeführt werden:
Durch eine Aminolyse eines substituierten Benzols der Formel (la) 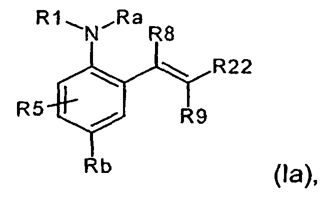 worin Ra für eine geeignete Schutzgruppe, wie sie zum Beispiel in Organic Synthesis, Kapitel 7, "Protection for the Amino Group", Seite 309 ff., Wiley Interscience, 1991 beschrieben wird, steht; **Rb** die Bedeutung NR1 Ra oder NR3R4 hat und **R22** für eine Carbonsäuregruppe, eine Carbonsäurechloridgruppe, eine Carbonsäureestergruppe oder eine Carbonsäureanhydridgruppe steht,
   mit einem Amin der Formel NHR6R7,
   wobei **R1, R3, R4, R5, R6, R7, R8** und **R9** die in Formel (I) genannte Bedeutung haben,
   und anschliessende Abspaltung der Schutzgruppe;

Die erfindungsgemäßen 3-(2,5-Diaminophenyl)-acrylamid-Derivate der Formel (I) sind in Wasser gut löslich und ermöglichen Färbungen mit hoher Farbintensität und ausgezeichneter Farbechtheit, insbesondere was die Lichtechtheit, Waschechtheit und Reibeechtheit anbetrifft.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein 3-(2,5-Diaminophenyl)-acrylamid-Derivat der Formel (I) enthalten.

Das 3-(2,5-Diaminophenyl)-acrylamid-Derivat der Formel (I) ist in dem erfindungsgemäßen Färbemittel in einer Menge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Kupplersubstanzen kommen vorzugsweise N-(3-Dimethylaminophenyl)-harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxypyridin, 3,5-Diamino-2,6-dimethoxy-pyridin, 1,3-Diamino-benzol, 2,4-Diamino-1-(2-hydroxy-ethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxy-ethyl)amino-toluol, 4-Hydroxyindol, 3-Dimethylamino-phenol, 3-Diethyl-amino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthatin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion. N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxy-ethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol; 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)aminoJ-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)aminol-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion in Betracht.

Obwohl die vorteilhaften Eigenschaften der hier beschriebenen 3-(2,5-Diaminophenyl)-acrylamid-Derivate der Formel (I) es nahelegen, diese als alleinige Entwicklersubstanz zu verwenden, ist es selbstverständlich auch möglich, die 3-(2,5-Diaminophenyl)-acrylamid-Derivate der Formel (I) gemeinsam mit bekannten Entwicklersubstanzen, wie zum Beispiel 1,4-Diaminobenzol, 2,5-Diaminotoluol, 1-(2,5-Diamino-phenyl)ethanol, 2-(2,5-Diaminophenyl)ethanol, 4-Aminophenol und seinen Derivaten, beispielsweise 4-Amino-3-methyl-phenol, 4,5-Diamino-pyrazolderivaten, beispielsweise 4,5-Diamino-1-(2-hydroxyethyl)-pyrazol, 4,5-Diamino-1-benzyl-pyrazol und 4,5-Diamino-1-(4-methylbenzyl)-pyrazol, oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuss oder Unterschuss vorhanden sind.

Weiterhin kann das erfindungsgemäße Färbemittel zusätzlich andere Farbkomponenten, beispielsweise 6-Amino-2-methylphenol und 2-Amino-5-methylphenol, sowie femer übliche direktziehende Farbstoffe, zum Beispiel Triphenylmethanfarbstoffe wie 4-[(4'-aminophenyl)-(4'-imino-2",5"-cyclohexadien-1 "-yliden)-methyl]-2-methylaminobenzol-monohydrochlorid (C.l. 42 510) und 4-[(4'amino-3'-methyl-phenyl)-(4"-imino-3"-methyl-2",5"cyclohexadien-1 "-yliden)-methyl]-2-methyl-aminobenzol monohydrochlorid (C.l. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5hydroxynaphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Färbemittel mit einem Gehalt an 3-(2,5-Diaminophenyl)-acrylamid-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften der Färbemittel gemäß der vorliegenden Anmeldung zeigen sich weiterhin darin, daß diese Mittel eine Anfärbung von ergrauten, chemisch nicht vorgeschädigten Keratinfasern, insbesondere menschlichen Haaren, problemlos und mit guter Deckkraft ermöglichen.

Die nachfolgenden Beispiele sollen den Gegenstand der Erfindung näher erläutern, ohne ihn darauf zu beschränken.

### Beispiele

### I. Herstellungsbeispiele

### Beispiele 1: Synthese von 3-(2,5-Diaminophenyl)-acrylamid-Derivaten der Formel (I) (Allgemeine Synthesevorschrift)

### A. Synthese von (2-Brom-4-tert-butoxycarbonylamino-phenyl)-carbaminsäure tert-butylester

15,65 g (0,07 mol) Brom-p-phenylendiamin-Hydrochlorid und 32,7 g (0,15 mol) Di-tert.-butyl-dicarbonat werden in einer Mischung aus 250 ml 2n Natriunhydroxid und 250 ml Trifluortoluol gelöst und auf 45 °C erwärmt. Die Reaktionmischung wird 3 Tage lang gerührt. Schrittweise werden noch insgesamt 30 g (0,14 mol) Di-tert.-butyl-dicarbonat zugegeben. Anschliessend wird die organische Schicht abgetrennt und die wässrige Phase noch zweimal mit 100 ml Dichlormethan extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 18,6 g (82 % der Theorie) 2,5-tert.-Butyloxycarbonylaminobrombenzol mit einem Schmelzpunkt von 130 °C erhalten.

### B. Synthese von N-(4-tert.Butyloxycarbonylamino-2-formyl-phenyl)-carbaminsäure-tert.butylester

3,3 g (0,01 mol) (2-Brom-4-tert-butoxycarbonylamino-phenyl)-carbaminsäure tert-butylester aus Stufe **A** werden unter Argon in 100 ml wasserfreiem Tetrahydrofuran gelöst. Schrittweise werden sodann 17 ml (0,03 mol) einer 1,6 molaren etherischen Methyllithiumlösung zugegeben. Die Reaktionsmischung wird auf -20 °C gekühlt und schrittweise mit 7 ml (0,01 mol) einer 1,5 molaren tert.-Butyllithiumlösung versetzt. Nach beendeter Zugabe wird die Lösung noch 30 Minuten lang bei -20 °C gerührt. Anschliessend werden 1,2 g (0,02 mol) Dimethylformamid zugegeben und die Reaktionmischung wird eine Stunde lang bei -20 °C gerührt. Nach langsamer Erwärmung auf Raumtemperatur wird die Reaktionsmischung mit Wasser hydrolisiert und dann auf Diethylether gegossen. Die wässerige Phase wird sodann mit Diethylether extrahiert und die organische Phase mit Magnesiumsulfat getrocknet. Das Lösungsmittel wird am Rotationsverdampfer abdestilliert und der Rückstand an Kieselgel mit Petrolether/Essigsäureethylester (9:1) gereinigt.

### C. Synthese von 3-(2,5-Bis-tert-butoxycarbonylamino-phenyl)-acrylsäuremethylester

9,5 g (0,03mol) von N-(4-tert.-Butyloxycarbonylamino-2-formyl-phenyl)-carbaminsäure-tert.-butylester aus Stufe **B** werden in 70 ml Tetrahydrofuran gelöst und wird mit 11,9 g (0,036 mol) Methoxycarbonylmethylentriphenylphosphoran versetzt. Die Reaktionsmsichung wird 6 Stunden lang bei Raumtemperatur gerührt. Das Reaktionsgemisch wird sodann auf Wasser gegossen und mit Essigsäureethylester extrahiert. Anschliessend wird die organische Phase mit einer gesättigten wässrigen NaCl-Lösung gewaschen, über Natriumsulfat getrocknet und nach Filtration eingeengt. Die Flash-Chromatographie des Rohproduktes an Kieselgel mit Hexan/Essigsäureethylester ergibt 10,2 g (87 % der Theorie).
¹H-NMR (300 MHz, CDCl₃): δ = 7,8 (d, 1 H); 7,69 (d, 1 H); 7,6 (br, 1 H); 7,21 (dd, 1 H); 6,50 (br, 1 H); 6,40 (d, 1 H); 6,35 (br, 1H); 3,8 (s, -O-CH₃); 1,51 (s, 18H)

### D. Synthese von 3-(2,5-Bis-tert-butoxycarbonylamino-phenyl)-acrylsäure

Zu einer Lösung von 7,1g (0,018 mol) von 3-(2,5-Bis-tert-butoxycarbonylamino-phenyl)-acrylsäuremethylester aus Stufe **C** in 500 ml Tetrahydrofuran und 300 ml Wasser wird bei 0 °C 2,53 g (0,06 mol) Lithiumhydroxid-Monohydrat zugegeben. Das Gemisch wird 48 Stunden bei Raumtemperatur gerührt, wobei jeweils nach 6 und 24 Stunde erneut 2,53 g (0,06 mol) Lithiumhydroxid-Monohydrat zugegeben werden. Das Reaktionsgemisch wird anschliessend in eine Phosphat-Puffer-Lösung (pH 7,0) gegossen und mit Essigsäureethylester extrahiert. Die organische Phase wird sodann mit einer gesättigten wässrigen NaCl-Lösung gewaschen und über Natriumsulfat getrocknet. Die organische Phase wird bis zur einsetzenden Niederschlagsbildung partiell eingeengt unnd mit Hexan versetzt. Der Niederschlag wird abfiltriert und mit 50 ml Hexan nachgewaschen.
Es werden 5 g (73% der Theorie) 3-(2,5-Bis-tert-butoxycarbonylaminophenyl)-acrylsäure erhalten.
¹H-NMR (300 MHz, DMSO-D₆): δ = 12,53 (br, 1 H); 9,38 (d; 1 H); 8,89 (s, 1 H); 7,79 (br, 1 H); 7,68 (d, 1 H); 7,42 (d, 1 H); 7,18 (d, 1 H); 6,21 (d, 1H); 1,48 (s, 9H); 1,43 (s, 9H)

### E. Synthese von 3-(2,5-Diamino-phenyl)-acrylamiden

Eine Mischung von 0,07g (0,185 mmol) von 3-(2,5-Bis-tert-butoxycarbonylamino-phenyl)-acrylsäure, 0,037g (0,24 mmol) N-Hydroxybenzotriazol-Hydrat, sowie 0,043 g (0,22 mmol) N-(3-Dimethylaminopropyl)-N'-ethyl-carbodiimid-Hydrochlorid wird in Dichlormethan vorgelegt, mit dem entsprechenden Amin (0,22 mmol) sowie mit 0,047 g N-Ethyldiisopropylamin versetzt und 12 Stunde bei Raumtemperatur geschüttelt.

Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit einem geeigneten Eluiermittel (z.B. Petrolether/Essigsäureethylester oder Dichlormethan/Methanol) gereinigt. Das so erhaltene Produkt wird in 4 ml Ethanol auf 50 °C erwärmt. Anschließend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.
**a.** N-(4-Aminophenyl)-3-(2,5-diaminophenylkacrylamid*HCl
   Verwendetes Amin: 4-Amino-phenyl-carbaminsäure-tert.butylester
   Masspektrum: MH⁺ 269 (100)
**b.** 3-(2,5-Diaminophenyl)-N-ethyl-acrylamid*HCl
   Verwendetes Amin: Ethylamin
   Masspektrum: MH⁺ 206 (100)
**c.** 3-(2,5-Diaminophenyl)-N-furan-2-ylmethyl-acrylamid*HCl
   Verwendetes Amin: Furfurylamin
   Masspektrum: MH⁺ 258 (100)
**d.** N-(4-Amino-2(3)-methylphenyl)-3-(2,5-diamino-phenyl)-acrylamid *HCl
   Verwendetes Amin: (4-Amino-2-methyl-phenyl)-carbaminsäure-tert.butylester und (4-Amino-3-methyl-phenyl)-carbaminsäure-tert.butylester
   Masspektrum: MH⁺ 283 (100)
e. N-Allyl-3-(2,5-diaminophenyl)-acrylamid*HCl
   Verwendetes Amin: Allylamin
   Masspektrum: MH⁺ 218 (100)
**f**. 3-(2,5-Diaminophenyl)-N-propyl-acrylamid*HCl
   Verwendetes Amin: Propylamin
   Masspektrum: MH⁺ 220 (42)
**g**. N-[4-Amino-2(3)-(2-hydroxyethyl)-phenyl]-3-(2,5-d)aminophenyl)-acrylamid*HCl
   Verwendetes Amin: (4-Amino-2-(2-hydroxyethyl)-phenyl)-carbaminsäure-tert.butylester und (4-Amino-3-(2-hydroxyethyl)-phenyl)-carbaminsäure-tert.butylester
   Masspektrum: MH⁺ 313 (100)
**h.** N-Cyclopropyl-3-(2,5-diarninophenyl)-acrylamid*HCl
   Verwendetes Amin: Cyclopropylamin
   Masspektrum: MH⁺ 218 (68)
i. N-[5-Amino-2(4)-(2-hydroxyethoxy)-phenyl]-3-(2,5-diaminophenyl)-acrylamid*HCl
   Verwendetes Amin: [3-Amino-4-(2-hydroxyethoxy)-phenyl]-carbaminsäure-tert.butylester und [3-Amino-6-(2-hydroxyethoxy)-phenyl]-carbaminsäure-tert.butylester
   Masspektrum: MH⁺ 392 (100)
**j**. 3-(2,5-Diaminophenyl)-N-methyl-acrylamid*HCl
   Verwendetes Amin: Methylamin
   Masspektrum: MH⁺ 192 (80)
**k.** 3-(2,5-Diaminophenyl)-N-isopropyl-acrylamid*HCl
   Verwendetes Amin: Isopropylamin
   Masspektrum: MH⁺ 220 (100)
**I.** 3-(2,5-Diaminophenyl)-N-(2-hydroxy-1-methylethyl)-acrylamid *HCl
   Verwendetes Amin: 2-Amino-propanol
   Masspektrum: MH⁺ 236 (100)
**m.** 3-(2,5-Diaminophenyl)-N-methoxy-N-methyl-acrylamid*HCl
   Verwendetes Amin: N,O-Dimethylhydroxylamin*HCl
   Masspektrum: MH⁺ 222 (92)
n. 3-(2,5-Diaminophenyl)-N-[2-(3H-imidazol-4yl)-ethyl]-acrylamid*HCl
   Verwendetes Amin: Histamin
   Masspektrum: MH⁺ 272 (100)
o. 3-(2,5-Diaminophenyl)-1-pyrrolidin-1-yl-propenon*HCl
   Verwendetes Amin: Pyrrolidin
   Masspektrum: MH⁺ 232 (100)
**p.** N-{4-[Bis-(2-hydroxyethyl)-amino]-phenyl}-3-(2,5-diaminophenyl)-acrylamid*HCl
   Verwendetes Amin: 4-Bis-(2-hydroxyethyl)-amino-anilin
   Masspektrum: MH⁺ 357 (100)
**q.** 3-(2,5-Diaminophenyl)-1-(3-hydroxy-pyrrolidin-1-yl)-propenon*HCl
   Verwendetes Amin: 3-Pyrrolidinol
   Masspektrum: MH⁺ 248 (100)
**r.** N-(3-Chlor-2-hydroxy-5-nitro-phenyl)-3-(2,5-diaminophenyl)-acrylamid *HCl
   Verwendetes Amin: 2-Amino-4-nitro-6-chlor-phenol
   Masspektrum: MH⁺ 349 (100)
**s.** 3-(2,5-Diaminophenyl)-N-(4-hydroxyphenyl)-acrylamid*HCl
   Verwendetes Amin: 4-Aminophenol
   Masspektrum: MH⁺ 270 (100)
**t.** 3-(2,5-Diaminophenyl)-N-(tetrahydrofuran-2-ylmethyl)-acrylamid*HCl
   Verwendetes Amin: Tetrahydrofurfurylamin
   Masspektrum: MH⁺ 262 (100)
**u.** 3-(2,5-Diaminophenyl)-N-(2-hydroxy-1-hydroxymethyl-ethyl)-acrylamid*HCl
   Verwendetes Amin: 3-Amino-1,2-propandiol
   Masspektrum: MH⁺ 252 (100)
**v.** N-(2-Aminoethyl)-3-(2,5-diaminophenyl)-acrylamid*HCl
   Verwendetes Amin: Ethylendiamin
   Masspektrum: MH⁺ 221 (100)
**w.** 3-(2,5-Diaminophenyl)-N-(3-imidazol-1-yl-propyl)-acrylamid*HCl
   Verwendetes Amin: 1-(3-Aminopropyl)-imidazol
   Masspektrum: MH⁺ 286 (100)
**x.** 3-(2,5-Diaminophenyl)-1-(2-hydroxymethyl-pyrrolidin-1-yl)-propenon*HCl
   Verwendetes Amin: Prolinol
   Masspektrum: MH⁺ 262 (100)
**y**. 3-(2,5-Diaminophenyl)-N-(2-methoxyethyl)-acrylamid*HCl
   Verwendetes Amin: 2-Methoxy-ethylamin
   Masspektrum: MH⁺ 236 (100)
**z.** 3-(2,5-Diamino-phenyl)-1-morpholin-4-yl-propenon HCl
   Verwendetes Amin: Morpholin
   Masspektrum: MH⁺ 248 (62)
**aa.** N-[4-Amino-2-(2-hydroxyethyl)-2H-pyrazol-3-yl]-3-(2,5-diaminophenyl)-acrylamid*HCl
   Verwendetes Amin: 4,5-Diamino-1-(2-hydroxyethyl)pyrazol
   Masspektrum: MH⁺ 303 (100)
**ab.** 3-(2,5-Diaminophenyl)-N-(1-hydroxymethylpropyl)-acrylamid*HCl
   Verwendetes Amin: 2-Amino-1-butanol
   Masspektrum: MH⁺ 250 (100)
**ac.** 3-(2,5-Diaminophenyl)-1-(4-hydroxy-piperidin-1-yl)-propenon*HCl
   Verwendetes Amin: 4-Hydroxy-piperidin
   Masspektrum: MH⁺ 262 (100)
**ad.** N-(2-Acetylamino-ethyl)-3-(2,5-diaminophenyl)-acrylamid*HCl
   Verwendetes Amin: N-Acetylethylendiamin
   Masspektrum: MH⁺ 263 (100)
**ae.** 3-(2,5-Diaminophenyl)-N-(2-morpholin-4-yl-ethyl)-acrylamid*HCl
   Verwendetes Amin: 4-(2-Ethylamino)-morpholin
   Masspektrum: MH⁺ 291 (100)
**af.** 3-(2,5-Diaminophenyl)N-[3-(2-oxo-pyrrolidin-1-yl)-propyl]-acrylamid*HCl
   Verwendetes Amin: 1-(3-aminopropyl)-2-pyrrolidon
   Masspektrum: MH⁺ 303(100)
**ag.** 2-[3-(2,5-Diaminophenyl)-acryloylaminol-3-methyl-buttersäure*HCl
   Verwendetes Amin: α-Amino-isovaleriansäure
   Masspektrum: MH⁺ 278 (88)
**ah.** [3-(2,5-Diaminophenyl)-acryloylamino]-essigsäure*HCl
   Verwendetes Amin: Glycin
   Masspektrum: (MH⁺ + CH₃CN) 277 (100)
**ai.** 3-(2,5-Diaminophenyl)-N-[2-(5-nitro-pyridin-2-ylamino)-ethyl]-acrylamid*HCl
   Verwendetes Amin: 2-Amino-5-nitro-pyridin
   Masspektrum: MH⁺ 343 (100)
**ai.** 3-(2,5-Diaminophenyl)-N-(2-(4-sulfamoyl-phenyl)ethyl]-acrylamid*HCl
   Verwendetes Amin: 4-(2-Amino-ethyl)-benzolsulfonamid
   Masspektrum: MH⁺ 361 (32)
**ak.** N-[5-Chlor-4-(2-hydroxyethylamino)-2-nitro-phenyl]-3-(2,5-diaminophenyl)-acrylamid*HCl
   Verwendetes Amin: 5-Chlor-4-(2-hydroxyethylamino)-2-nitro-anilin
   Masspektrum: MH⁺ 392 (28)
**al.** 3-(2,5-Diaminophenyl)-N-pyridin-2-yl-acrylamid*HCl
   Verwendetes Amin: 2-Amino-pyridin
   Masspektrum: MH⁺ 255 (100)
**am.** N-[3-(2,5-Diaminophenyl)-prop-2-enoyl]-L-glutaminsäure-trifluoracetat
   Verwendetes Amin: L-Glutaminsäure
   Masspektrum: MH⁺ 308 (100)
**an.** N2-[3-(2,5-Diaminophenyl)-prop-2-enoyl]-L-glutamin-trifluoracetat
   Verwendetes Amin: L-Glutamin
   Masspektrum: MH⁺ 307 (100)
**ao.** 3-(2,5-Diaminophenyl)-N-(3-hydroxy-4-methyl-phenyl)-acrylamid*HCl
   Verwendetes Amin: 5-Amino-2-methyl-phenol
   Masspektrum: MH⁺ 284 (100)
**ap.** N-(3-Amino-phenyl)-3-(2,5-diaminophenyl)-acrylamid*HCl
   Verwendetes Amin: (3-Amino-phenyl)-carbaminsäure-tert.butylester
   Masspektrum: MH⁺ 269 (100)
**aq.** 3-(2,5-Diaminophenyl)-N-(3-hydroxy-5-hydroxymethyl-2-methyl
   pyridin-4-ylmethyl)-acrylamid*HCl
   Verwendetes Amin: 4-Aminomethyl-5-hydroxymethyl-2-methyl-pyridin-3-ol Masspektrum: MH⁺ 329 (100)
**ar.** 3-(2,5-Diaminophenyl)-N-(2-hydroxy-5-nitro-phenyl)-acrylamid*HCl
   Verwendetes Amin: 2-Amino-4-nitro-phenol
   Masspektrum: MH⁺ 315 (100)
as. 1-[3-(2,5-Diaminophenyl)-acryloyl]-pyrrolidine-2-carbonsäureamid*HCl
   Verwendetes Amin: Prolinamid
   Masspektrum: MH⁺ 275 (100)
at. 3-(2,5-Diaminophenyl)-1-(3-hydroxy-piperidin-1-yl)-propenon*HCl
   Verwendetes Amin: 3-Hydroxy-piperidin
   Masspektrum: MH⁺ 262 (100)
av. N-(1-Carbamoyl-2-hydroxyethyl)-3-(2,5-diaminoehenyl)-acrylamid*HCl
   Verwendetes Amin: 2-Amino-3-hydroxy-propionamid
   Masspektrum: MH⁺ 265 (100)
aw. N-[3-(2,5-Diaminophenyl)-prop-2-enoyl]-L-asparaginsäure-trifluoracetat
   Verwendetes Amin: Asparaginsäure
   Masspektrum: MH⁺ 294 (100)
**ax.** N2-[3-(2,5-diaminophenyl)-prop-2-enoyl]-L-asparagin-trifluoracetat
   Verwendetes Amin: Asparagin
   Masspektrum: MH⁺ 293 (100)
**ay**. N-Benzo[1,3]dioxol-5yl-3-(2,5-diaminophenyl)-acrylamid*HCl
   Verwendetes Amin: Benzo[1,3]dioxol-5-ylamin
   Masspektrum: MH⁺ 298 (100)
**az.** N-[3-(2,5-Diaminophenyl)-prop-2-enoyl]-L-leucin*HCl
   Verwendetes Amin: L-Leucin
   Masspektrum: MH⁺ 334 (100)

### Beispiele 2 bis 36: Haarfärbemittel

Es werden Haarfärbelösungen der folgenden Zusammensetzung hergestellt:

| | |
|---|---|
| 1,25 mmol | Entwicklersubstanz der Formel (I) gemäß Tabelle 1 |
| 1,25 mmol | Kupplersubstanz gemäß Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefasst.

**Tabelle 1:**

| **Beispiel Nr.** | **Entwickler-substanz der Formel (I)** | **Kupplersubstanz** | | | |
|---|---|---|---|---|---|
| | | **I.** | **II.** | **III.** | **IV.** |
| | | **1,3-Di-hydroxy-benzol** | **1,3-Diamino-4-(2-hydroxy-ethoxy)-benzol-sulfat** | **5-Amino-2-methylphenol** | **1-Naphtol** |
| **2.** | Gemäß Beispiel **1a** | mittel-blond | dunkelgrau | purpur | grau |
| **3.** | gemäß Beispiel **1b** | mittel-blond | blau | purpur | grau |
| 4. | gemäß Beispiel **1c** | mittel-blond | blau-grau | purpur | grau |
| **5.** | gemäß Beispiel **1d** | mittel-blond | blau-grau | purpur | grau |
| **6.** | gemäß Beispiel **1e** | mittel-blond | blau-grau | purpur | grau |
| **7.** | gemäß Beispiel **1f** | mittel-blond | blau-grau | purpur | grau |
| **8.** | gemäß Beispiel **1g** | mittel-blond | blau-grau | purpur | grau |
| **9.** | gemäß Beispiel **1h** | mittel-blond | blau-grau | purpur | grau |
| **10.** | gemäß Beispiel **1i** | mittel-blond | blau-grau | purpur | grau |
| **11.** | gemäß Beispiel **1j** | mittel-blond | blau-grau | purpur | grau |
| **12.** | gemäß Beispiel **1k** | mittel-blond | blau-grau | purpur | grau |
| 13. | gemäß Beispiel **1l** | mittel-blond | blau-grau | purpur | grau |
| **14.** | gemäß Beispiel **1m** | mittel-blond | blau-grau | purpur | grau |
| **15.** | gemäß Beispiel **1n** | mittel-blond | blau-grau | purpur | grau |
| **16.** | gemäß Beispiel **1o** | blond | blau-grau | purpur | grau |
| **17.** | gemäß Beispiel **1p** | mittel-blond | blau-grau | purpur | grau |
| **18.** | gemäß Beispiel **1q** | blond | blau-grau | purpur | grau |
| **19.** | gemäß Beispiel **1r** | mittel-bond | grün | braun | grün |
| **20.** | gemäß Beispiel **1s** | blond | braun | rotbraun | mittelbraun |
| **21.** | gemäß Beispiel **1t** | mittel-blond | blau-grau | purpur | grau |
| 22. | gemäß Beispiel **1u** | blond | blau-grau | purpur | grau |
| 23. | gemäß Beispiel **1v** | mittel-blond | blau-grau | purpur | grau |
| **24.** | gemäß Beispiel **1w** | blond | hell-blau | hell-purpur | hell-grau |
| **25.** | gemäß Beispiel **1x** | blond | hell-blau | hell-purpur | hell-grau |
| **26.** | gemäß Beispiel **1y** | blond | hell-blau | hell-purpur | hell-grau |
| **27.** | gemäß Beispiel **1z** | blond | hell-blau | hell-purpur | hell-grau |
| **28.** | gemäß Beispiel **1aa** | hellrot | dunkelviolet | rot | violet |
| **29.** | gemäß Beispiel **1ab** | blond | hell-blau | hell-purpur | hell-grau |
| **30.** | gemäß Beispiel **1ac** | blond | hell-blau | hell-purpur | hell-grau |
| **31.** | gemäß Beispiel **1ad** | hell-blond | hell-blau | hell-purpur | hell-grau |
| **32.** | gemäß Beispiel **1ae** | hell-blond | hell-blau | hell-purpur | hell-grau |
| **33.** | gemäß Beispiel **1af** | hell-blond | hell-blau | hell-purpur | hell-grau |
| **34.** | gemäß Beispiel **1ag** | hell-blond | hell-blau | hell-purpur | hell-grau |
| **35.** | gemäß Beispiel **1ah** | hell-blond | hell-blau | hell-purpur | hell-grau |
| **36.** | gemäß Beispiel **1ai** | hell-blond | hell-blau | hell-purpur | hell-grau |

### Beispiele 37 bis 58: Haarfärbemittel

Es werden Haarfärbelösungen der folgenden Zusammensetzung hergestellt:

| | |
|---|---|
| X g | 3-(2,5-Diaminophenyl)-acrylamid-Derivat der Formel (I) (Entwicklersubstanz **E1, E2, E3** gemäß Tabelle 2) |
| U g | Entwicklersubstanz **E8** bis **E15** gemäß Tabelle 2 |
| Y g | Kupplersubstanz **K12** bis **K33** gemäß Tabelle 3 |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässsrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 4 zusammengefasst.

### Beispiele 59 bis 64: Haarfärbemittel

Es werden cremeförmige Farbträgermassen der folgenden Zusammensetzung hergestellt:

| | |
|---|---|
| X g | 3-(2,5-Diaminophenyl)-acrylamid-Derivat der Formel (I) (Entwicklersubstanz **E1, E2, E3** gemäß Tabelle 2) |
| U g | Entwicklersubstanz **E8** bis **E15** gemäß Tabelle 2 |
| Y g | Kupplersubstanz **K12** bis **K33** gemäss Tabelle 3 |
| Z g | 6-Chlor-2-ethylamino-4-nitro-phenot **(D2)** |
| 15,0 g | Cetylalkohol |
| 0,3 g | Ascorbinsäure |
| 3,5 g | Natriumlaurylalkoholdiglycolethersulfat, 28prozentige wässrige Lösung |
| 3,0 g | Ammoniak, 22prozentige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6prozentigen Wasserstoffperoxidlösung vermischt. Anschliessend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

Alle in der vorliegenden Anmeldung enthaltenen Prozentangaben stellen soweit nicht anders angegeben Gewichtsprozente dar.

**Tabelle 2:**

| **Entwicklersubstanzen** | |
|---|---|
| **E1** | 3-(2,5-Diaminophenyl)-N-ethyl-acrylamid*HCl |
| **E2** | N-Allyl-3-(2,5-diaminophenyl)-acrylamid*HC |
| **E3** | N-(4-Aminophenyl)-3-(2,5-diaminophenyl)-acrylamid*HCl |
| | |
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol*2HCl |
| **E12** | 4-Amino-phenol |
| **E14** | 4,5-Diamino-1-(2'-hydroxyethyl)-pyrazol-sulfat |
| **E15** | 2,5-Diaminotoluol-sulfat |

**Tabelle 3:**

| **Kupplersubstanzen** | |
|---|---|
| **K12** | 2-Amino-4-(2'-hydroxyethyl)amino-anisol-sulfat |
| **K13** | 1,3-Diamino-4-(2'-hydroxyethoxy)benzol-sulfat |
| **K21** | 3-Amino-phenol |
| **K22** | 5-Amino-2-methyl-phenol |
| **K23** | 3-Amino-2-chlor-6-methyl-phenol |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |

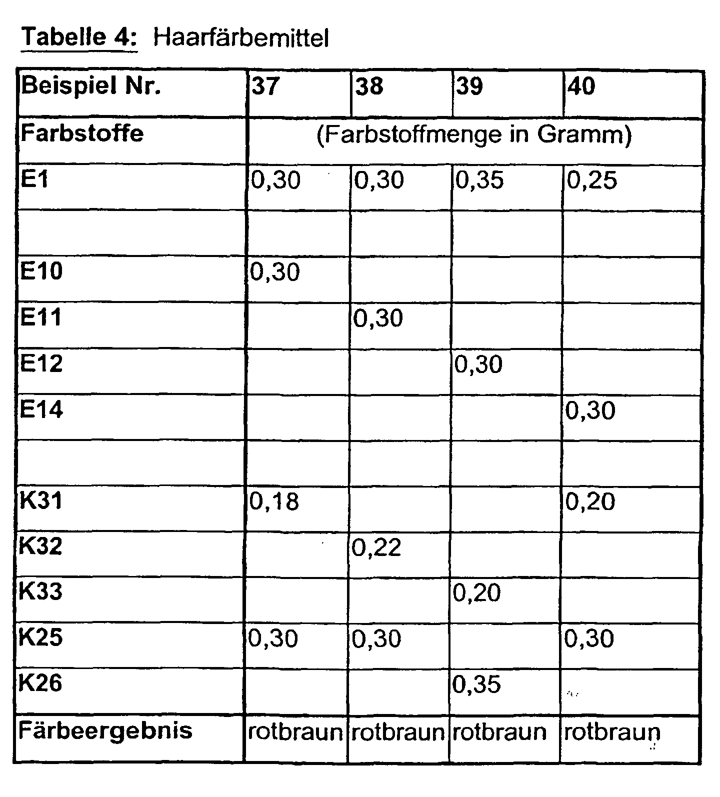

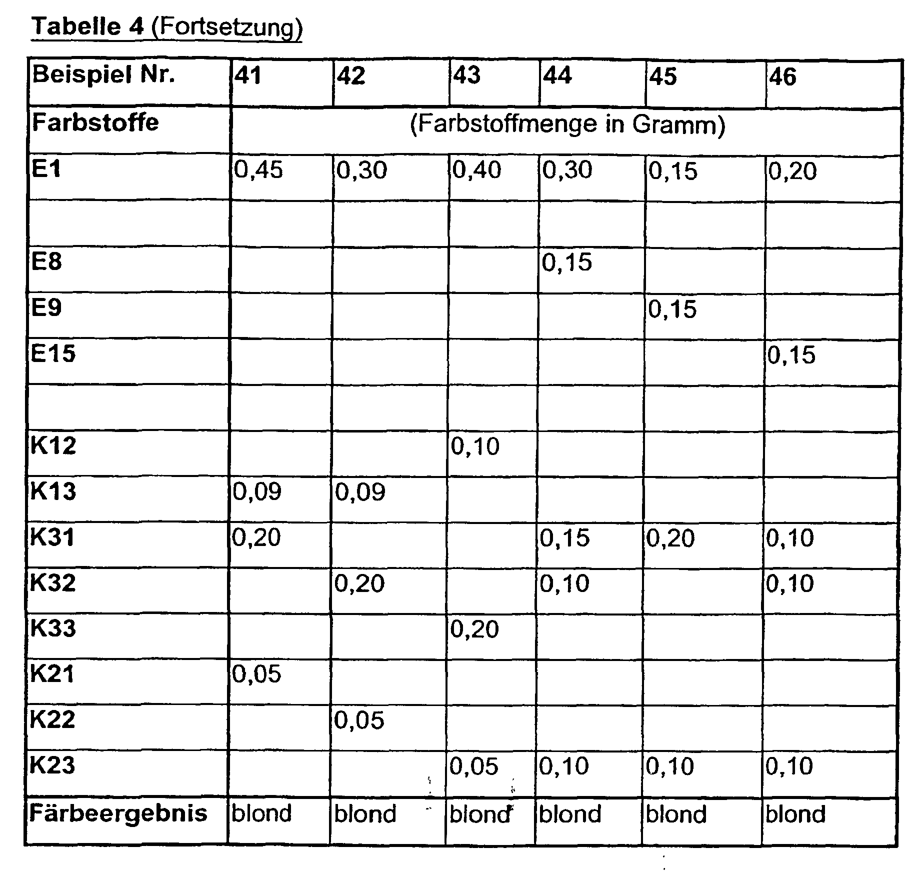

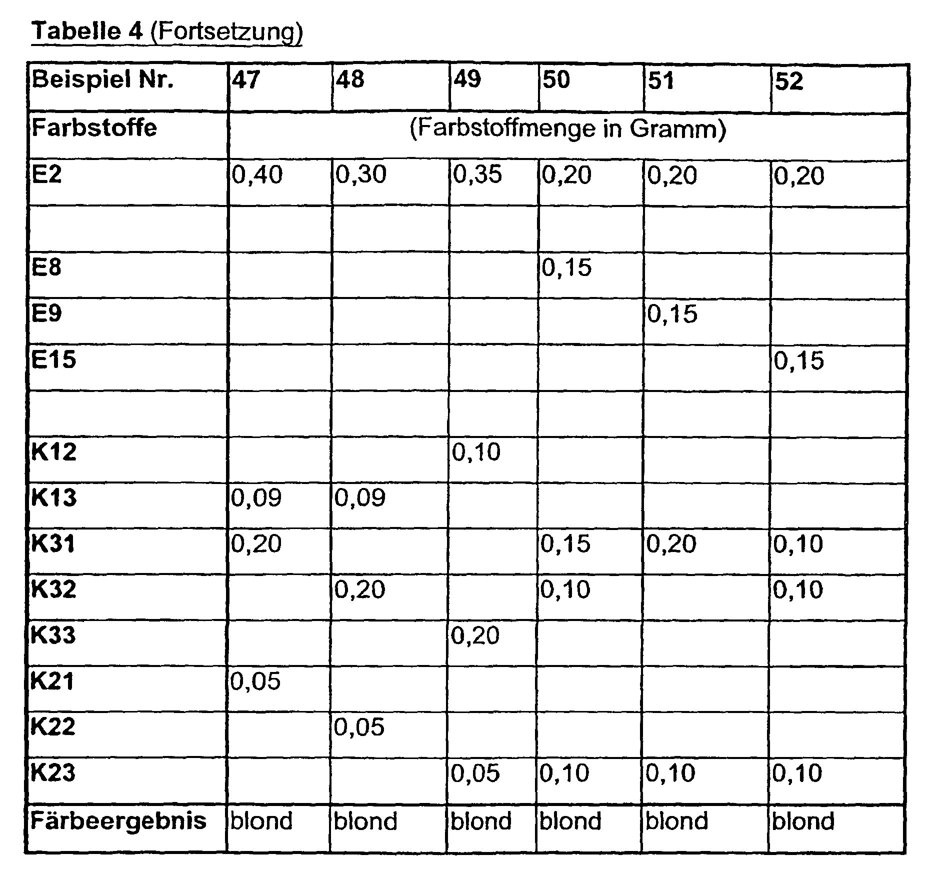

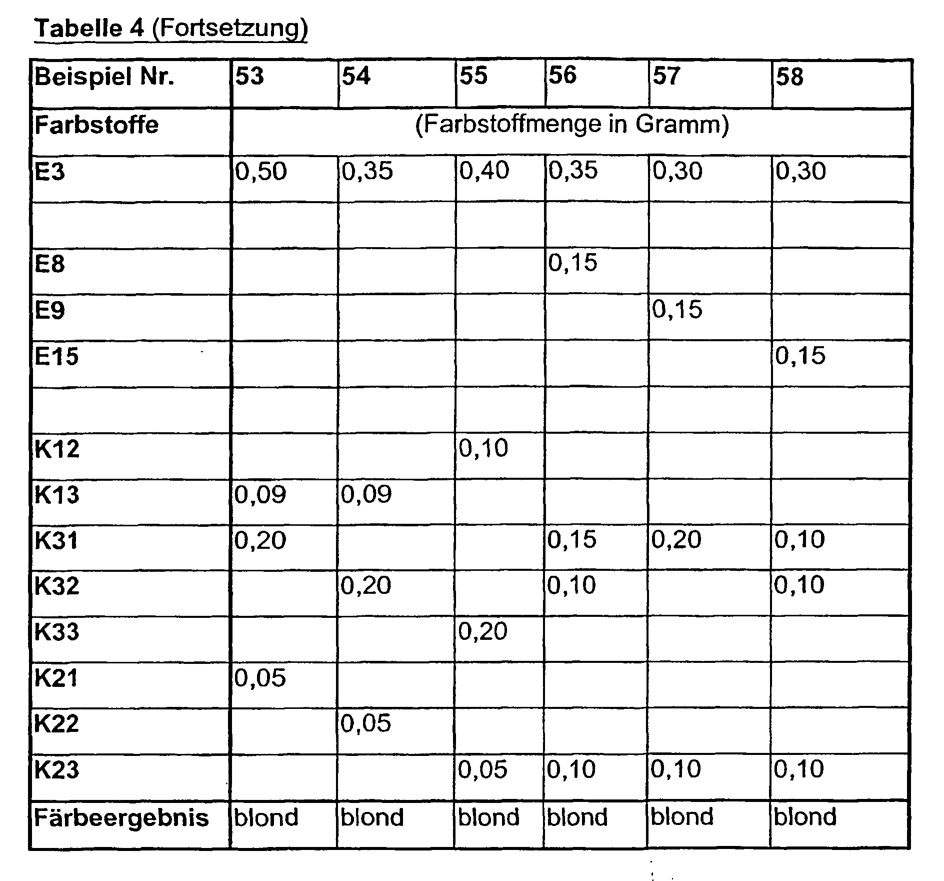

**Tabelle 5:**

| Haarfärbemittel | | | | | | |
|---|---|---|---|---|---|---|
| **Beispiel Nr.** | **59** | **60** | **61** | **62** | **63** | **64** |
| **Farbstoffe** | (Farbstoffmenge in Gramm) | | | | | |
| **E1** | 0,40 | 0,50 | | 0,80 | | 0,70 |
| **E2** | | | 0,40 | | 0,90 | |
| **E3** | | | | | | 1,00 |
| | | | | | | |
| **E9** | | 0,50 | | | | |
| **E15** | 0,50 | | 0,50 | | | |
| | | | | | | |
| **K12** | 0,10 | 0,10 | | 0,10 | 0,10 | 0,10 |
| **K23** | 0,10 | 0,10 | 0,05 | 0,10 | 0,10 | 0,10 |
| **K31** | 0,40 | 0,40 | 0,40 | 0,40 | 0,40 | 0,40 |
| | | | | | | |
| **D2** | 0,10 | 0,10 | 0,10 | 0,10 | 0,10 | 0,10 |
| **Färbeergebnis** | braun | braun | braun | braun | braun | braun |

## Patentansprüche

1. 3-(2,5-Diaminophenyl)-acrylamid-Derivate der allgemeinen Formel (I) oder deren physiologisch verträglichen, wasserlöslichen Salze, 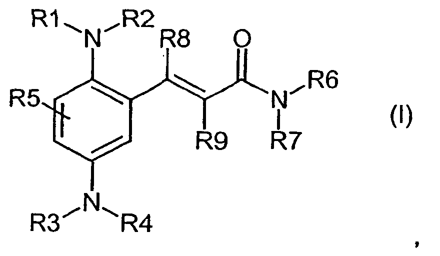 worin
**R1, R2, R3** und **R4** gleich oder verschieden sein können und unabhängig voneinander Wasserstoff, eine C₁-C₆-Alkylgruppe, eine C₂-C₄-Hydroxyalkylgruppe oder eine C₃-C₄-Dihydroxyalkylgruppe darstellen oder **R1** und **R2** beziehungsweise **R3** und **R4** einen viergliedrigen bis achtgliedrigen aliphatischen Ring bilden, wobei mindestens 2 der Reste **R1** bis **R4** Wasserstoff darstellen;
**R5** gleich Wasserstoff, einem Halogenatom, einer C₁-C₄-Alkylgruppe, einer C₁-C₄ -Hydroxyalkylgruppe oder einer C₁-C₄-Alkoxygruppe ist;
**R6** und **R7** gleich oder verschieden sein können und unabhängig voneinander gleich Wasserstoff, einer C₁-C₂-Alkoxygruppe, einer C₁-C₆-Alkylgruppe, einer ungesättigten C₃-C₆-Alkylgruppe, einer C₂-C₄-Hydroxyalkylgruppe, einer C₃-C₄-Dihydroxyalkylgruppe, einer C₂-C₄-Aminoalkylgruppe, einer C₂-C₄-Dimethylaminoalkylgruppe, einer C₂-C₄-Acetylaminoalkylgruppe einer C₂-C₄-Methoxyalkylgruppe, einer C₂-C₄-Ethoxyalkylgruppe, einer C₁-C₄-Cyanalkylgruppe, einer C₁-C₄-Carboxyalkylgruppe, einer C₂-C₄-Aminocarbonylalkylgruppe, einer Pyridylmethylgruppe, einer Furfurylgruppe, einer hydrierten Furfurylgruppe, einer substituierten Pyridylgruppe, einem Rest der Formel (II) 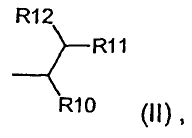 einem Rest der Formel (III) 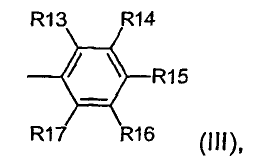 oder einem Rest der Formel (IV) 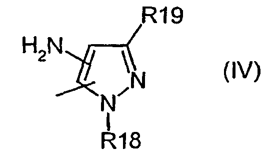 sind, oder **R6** und **R7** einen Ring der Formel 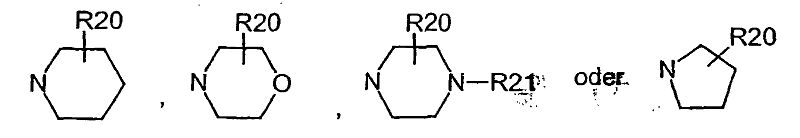 bilden;
**R8, R9** und **R21** gleich oder verschieden sein können und unabhängig voneinander gleich Wasserstoff, oder einer C₁-C₆ Alkylgruppe sind;
**R10** gleich Wasserstoff, einer Carboxygruppe, oder einer Aminocarbonylgruppe ist;
**R11** und **R12** gleich oder verschieden sein können und unabhängig voneinander gleich Wasserstoff, einer Hydroxygruppe, einer Aminocarbonylgruppe, einer Methylthiomethylgruppe, einem mit einer Phenylgruppe oder Hydroxygruppe substituierten Phenylrest oder einem Rest der Formel 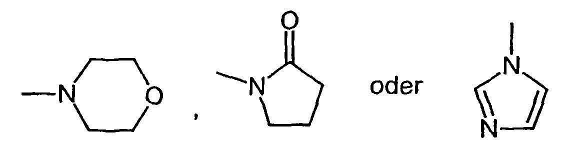 sind;
**R13, R14, R15, R16** und **R17** gleich oder verschieden sein können und unabhängig voneinander gleich Wasserstoff, einem Halogenatom, einer Cyanogruppe, einer Hydroxygruppe, einer C₁-C₄-Alkoxygruppe, einer C₁-C₄-Hydroxyalkoxygruppe, einer C₁-C₆-Alkylgruppe, einer C₁-C₄-Alkylthioethergruppe, einer Mercaptogruppe, einer Nitrogruppe, einer Aminogruppe, einer Alkylaminogruppe, einer Hydroxyalkylaminogruppe, einer Dialkylaminogruppe, einer Di(hydroxyalkyl)aminogruppe, einer (Dihydroxyalkyl)aminogruppe, einer (Hydroxyalkyl)alkylaminogruppe, einer Trifluormethangruppe, einer -C(O)H-Gruppe, einer -C(O)CH₃-Gruppe, einer -C(O)CF₃-Gruppe, einre -Si(CH₃)₃-Gruppe, einer C₁-C₄-Hydroxyalkylgruppe, einer C₂-C₄ Dihydroxyalkylgruppe sind, oder zwei nebeneinanderliegende Reste **R13** bis **R17** gemeinsam eine -O-CH₂-O-Brücke bilden;
**R18** gleich einer C₁-C₄-Alkylgruppe einer Benzylgruppe oder einer C₁-C₄-Hydroxyalkylgruppe ist;
**R19** gleich Wasserstoff oder einer C₁-C₆ Alkylgruppe ist;
**R20** gleich Wasserstoff, einer Hydroxygruppe, einer Carboxygruppe, einer Aminocarbonylgruppe oder einer Hydroxymethylgruppe ist.

2. 3-(2,5-Diaminophenyl)-acrylamid-Derivat nach Anspruch 1, **dadurch gekennzeichnet, dass** in der Formel (I) (i) eine oder mehrere der Restgruppen **R5, R8** und **R9** gleich Wasserstoff sind und/oder (ii) **R1, R2, R3** und **R4** gleich Wasserstoff sind und/oder (iii) **R6** gleich einer Methylgruppe, einer Methoxygruppe oder einer C₂-C₄-Hydroxyalkylgruppe und **R7** gleich einer C₂-C₄-Hydroxyalkylgruppe ist und/oder (iv) **R6** gleich Wasserstoff und **R7** gleich einer C₁-C₆-Alkylgruppe, einer ungesättigten C₃-C₆ -Alkylgruppe, einer C₂-C₄-Hydroxyalkylgruppe, einem Furfurylrest, einem substituierten Phenylrest oder einem subsituierten Pyrazoylrest ist, und/oder (v) **R6** und **R7** einen aliphatischen Ring der Formel 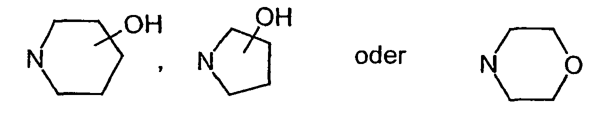 bilden.

3. 3-(2,5-Diaminophenyl)-acrylamid-Derivat nach Anspruch 1 oder 2, **dadurch gekennzeichnet, dass** es ausgewählt ist aus 3-(2,5-Diaminophenyl)-N-propyl-acrylamid, 3-(2,5-Diaminophenyl)-N-furan-2-ylmethylacrylamid, 3-(2,5-Diaminophenyl)-N-methoxy-N-methyl-acrylamid, 3-(2,5-Diaminophenyl)-N-(2-hydroxy-1-methylethyl)-acrylamid, N-Cyclopropyl-3-(2,5-diaminophenyl)-acrylamid, 3-(2,5-Diaminophenyl)-N-isopropyl-acrylamid, N-(4-Aminophenyl)-3-(2,5-diaminophenyl)-acrylamid, 3-(2,5-Diaminophenyl)-1-pyrrolidin-1-yl-propenon, N-[4-Amino-2-(2-hydroxyethyl)-2H-pyrazol-3-yl]-3-(2,5-diaminophenyl)-acrylamid, N-(4-Amino-2(3)-methylphenyl)-3-(2,5-diaminophenyl)-acrylamid, N-{4-[Bis-(2-hydroxyethyl)amino)-phenyl}-3-(2,5-diaminophenyl)-acrylamid, 3-(2,5-Diaminophenyl)-N-(4-hydroxyphenyl)-acrylamid, 3-(2,5-Diaminophenyl)-1-(3-hydroxy-pyrrolidin-1-yl)-propenon, N-[5-Amino-2(4)-(2-hydroxyethoxy)-phenyl]-3-(2,5-diaminophenyl)-acrylamid und 3-(2,5-Diamino-phenyl)-N-ethyl-acrylamid.

4. Mittel zum oxidativen Färben von Keratinfasern, auf der Basis einer Entwicklersubstanz-Kupplersubstanz-Kombination, **dadurch gekennzeichnet, dass** es als Entwicklersubstanz mindestens ein 3-(2,5-Diaminophenyl)-acrylamid-Derivat der Formel (I) nach einem der Ansprüche 1 bis 3 enthält.

5. Mittel nach Anspruch 4, **dadurch gekennzeichnet, dass** es das 3-(2,5-Diaminophenyl)-acrylamid-Derivat der Formel (I) in einer Menge von 0,005 bis 20 Gewichtsprozent enthält.

6. Mittel nach Anspruch 4 oder 5, **dadurch gekennzeichnet, dass** die Kupplersubstanz ausgewählt ist aus N-(3-Dimethylamino-phenyl)-harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxy-ethyl)amino)-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxy-ethoxy)-benzol, 1,3-Diamino-4-(2,3-dihydroxy-propoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxy-ethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino)-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxy-ethyl)amino-toluol, 4-Hydroxyindol, 3-Dimethylamino-phenol, 3-Diethyl-amino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxy-ethyl)amino]-4-methoxy-2-methyl-phenof, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion. N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxy-ethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxyyethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor 3,5-dihydroxy-4-methyl-benzol, 1,5-Dichlor-2,4-dihydroxy-benzol, 1,3-Dihydroxy-2-methy4-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

7. Mittel einem der Ansprüche 4 bis 6, **dadurch gekennzeichnet, dass** es zusätzlich zu dem 3-(2,5-Diaminophenyl)-acrylamid-Derivat der Formel (I) mindestens eine weitere Entwicklersubstanz aus der Gruppe bestehend aus 1,4-Diaminobenzol, 2,5-Diaminotoluol, 1-(2,5-Diamino-phenyl)ethanol, 2-(2,5-Diaminophenyl)ethanol, 4-Aminophenol und seinen Derivaten, 4,5-Diamino-pyrazolderivaten und Tetraaminopyrimidinen enthält.

8. Mittel nach einem der Ansprüche 4 bis 7, **dadurch gekennzeichnet, dass** die Entwicklersubstanzen und Kupplersubstanzen, bezogen auf die Gesamtmenge des Oxidationsfärbemittel, jeweils in einer Gesamtmenge von 0,005 bis 20 Gewichtsprozent enthalten sind.

9. Mittel nach einem der Ansprüche 4 bis 8 , **dadurch gekennzeichnet, daß** es zusätzlich mindestens einen direktziehenden Farbstoff enthält.

10. Mittel nach einem der Ansprüche 4 bis 9, **dadurch gekennzeichnet, daß** es einen pH-Wert von 6,5 bis 11,5 aufweist.

11. Mittel nach einem der Ansprüche 4 bis 10, **dadurch gekennzeichnet, dass** es ein Haarfärbemittel ist.

## Claims

1. 3-(2,5-Diaminophenyl)acrylamide derivatives of the general formula (I) or physiologically compatible water-soluble salts thereof, 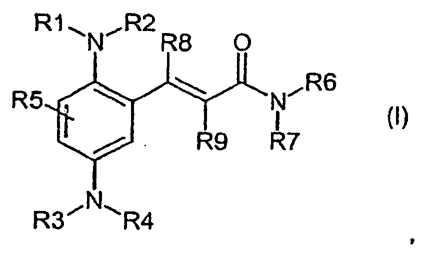 in which
**R1, R2, R3** and **R4** may be identical or different and, independently of one another, are hydrogen, a C₁-C₆-alkyl group, a C₂-C₄-hydroxyalkyl group or a C₃-C₄-dihydroxyalkyl group, or **R1** and **R2** or **R3** and **R4** form a four-membered to eight-membered aliphatic ring, where at least two of the radicals **R1** to **R4** are hydrogen;
**R5** is hydrogen, a halogen atom, a C₁-C₄-alkyl group, a C₁-C₄-hydroxyalkyl group or a C₁-C₄-alkoxy group;
**R6** and **R7** may be identical or different and, independently of one another, are hydrogen, a C₁-C₂-alkoxy group, a C₁-C₆-alkyl group, an unsaturated C₃-C₆-alkyl group, a C₂-C₄-hydroxyalkyl group, a C₃-C₄-dihydroxyalkyl group, a C₂-C₄-aminoalkyl group, a C₂-C₄-dimethylaminoalkyl group, a C₂-C₄-acetylaminoalkyl group, a C₂-C₄-methoxyalkyl group, a C₂-C₄-ethoxyalkyl group, a C₁-C₄-cyanoalkyl group, a C₁-C₄-carboxyalkyl group, a C₂-C₄-aminocarbonylalkyl group, a pyridylmethyl group, a furfuryl group, a hydrogenated furfuryl group, a substituted pyridyl group, a radical of the formula (II) 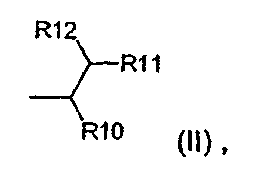 a radical of the formula (III) 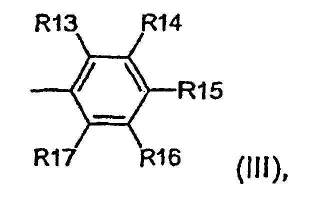 or a radical of the formula (IV) 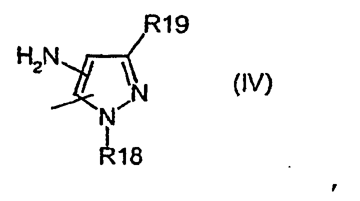 or **R6** and **R7** form a ring of the formula 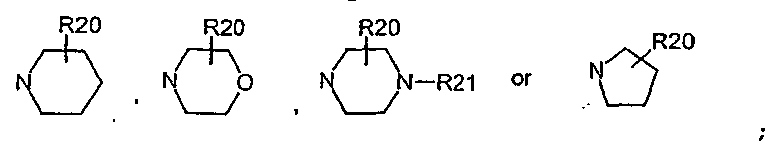 **R8, R9** and **R21** may be identical or different and, independently of one another, are hydrogen or a C₁-C₆-alkyl group;
**R10** is hydrogen, a carboxy group or an aminocarbonyl group;
**R11** and **R12** may be identical or different and, independently of one another, are hydrogen, a hydroxy group, an aminocarbonyl group, a methylthiomethyl group, a phenyl radical substituted by a phenyl group or hydroxy group, or a radical of the formula 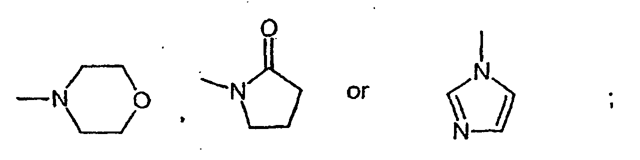 **R13, R14, R15, R16** and **R17** may be identical or different and, independently of one another, are hydrogen, a halogen atom, a cyano group, a hydroxy group, a C₁-C₄-alkoxy group, a C₁-C₄-hydroxyalkoxy group, a C₁-C₆-alkyl group, a C₁-C₄-alkyl thioether group, a mercapto group, a nitro group, an amino group, an alkylamino group, a hydroxyalkylamino group, a dialkylamino group, a di(hydroxyalkyl)amino group, a (dihydroxyalkyl)amino group, a (hydroxyalkyl)alkylamino group, a trifluoromethane group, a -C(O)H group, a -C (O) CH₃ group, a -C(O)CF₃ group, a -Si(CH₃)₃ group, a C₁-C₄-hydroxyalkyl group, a C₂-C₄-dihydroxyalkyl group, or two adjacent radicals **R13** to **R17** together form an -OCH2-O bridge;
**R18** is a C₁-C₄-alkyl group, a benzyl group or a C₁-C₄-hydroxyalkyl group;
**R19** is hydrogen or a C₁-C₆-alkyl group;
**R20** is hydrogen, a hydroxy group, a carboxy group, an aminocarbonyl group or a hydroxymethyl group.

2. 3-(2,5-Diaminophenyl)acrylamide derivative according to Claim 1, **characterized in that**, in the formula (I) (i) one or more of the radical groups **R5, R8** and **R9** are hydrogen and/or (ii) **R1, R2, R3** and **R4** are hydrogen and/or (iii) **R6** is a methyl group, a methoxy group or a C₂-C₄-hydroxyalkyl group and **R7** is a C₂-C₄-hydroxyalkyl group and/or (iv) **R6** is hydrogen and **R7** is a C₁-C₆-alkyl group, an unsaturated C₃-C₆-alkyl group, a C₂-C₄-hydroxyalkyl group, a furfuryl radical, a substituted phenyl radical or a substituted pyrazoyl radical, and/or (v) **R6** and **R7** form an aliphatic ring of the formula

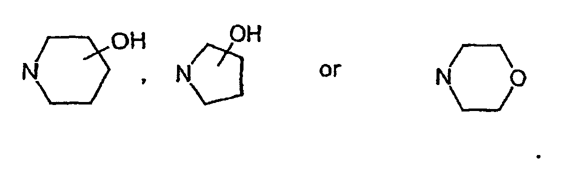

3. 3-(2,5-Diaminophenyl)acrylamide derivative according to Claim 1 or 2, **characterized in that** it is chosen from 3-(2,5-diaminophenyl)-N-propylacrylamide, 3-(2,5-diaminophenyl)-N-furan-2-ylmethylacrylamide, 3-(2,5-diaminophenyl)-N-methoxy-N-methylacrylamide, 3-(2,5-diaminophenyl)-N-(2-hydroxy-1-methylethyl)acrylamide, N-cyclopropyl-3-(2,5-diaminophenyl)acrylamide, 3-(2,5-diaminophenyl)-N-isopropylacrylamide, N-(4-aminophenyl)-3-(2,5-diaminophenyl)acrylamide, 3-(2,5-diaminophenyl)-1-pyrrolidin-1-ylpropenone, N-[4-amino-2-(2-hydroxyethyl)-2H-pyrazol-3-yl]-3-(2,5-diaminophenyl)acrylamide, N-(4-amino-2(3)-methylphenyl)-3-(2,5-diaminophenyl)acrylamide, N-{4-[bis(2-hydroxyethyl)amino]phenyl}-3-(2,5-, diaminophenyl)acrylamide, 3-(2,5-diaminophenyl)-N-(4-hydroxyphenyl)acrylamide, 3-(2,5-diaminophenyl)-1-(3-hydroxypyrrolidin-1-yl)propenone, N-[5-amino-2(4)-(2-hydroxyethoxy)phenyl]-3-(2,5-diaminophenyl)acrylamide and 3-(2,5-diaminophenyl)-N-ethylacrylamide.

4. Agent for the oxidative dyeing of keratin fibres, based on a developer substance-coupler substance combination, **characterized in that** it comprises, as developer substance, at least one 3-(2,5-diaminophenyl)acrylamide derivative of the formula (I) according to one of Claims 1 to 3.

5. Agent according to Claim 4, **characterized in that** it comprises the 3-(2,5-diaminophenyl)acrylamide derivative of the formula (I) in an amount of from 0.005 to 20 per cent by weight.

6. Agent according to Claim 4 or 5, **characterized in that** the coupler substance is chosen from N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione, N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl) phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

7. Agent according to one of Claims 4 to 6, **characterized in that**, in addition to the 3-(2,5-diaminophenyl)acrylamide derivative of the formula (I), it comprises at least one further developer substance from the group consisting of 1,4-diaminobenzene, 2,5-diaminotoluene, 1-(2,5-diaminophenyl)ethanol, 2-(2,5-diaminophenyl)ethanol, 4-aminophenol and its derivatives, 4,5-diaminopyrazole derivatives and tetraaminopyrimidines.

8. Agent according to one of Claims 4 to 7, **characterized in that** the developer substances and coupler substances, based on the total amount of the oxidation colouring agent, are in each case present in a total amount of from 0.005 to 20 per cent by weight.

9. Agent according to one of Claims 4 to 8, **characterized in that** it additionally comprises at least one direct dye.

10. Agent according to one of Claims 4 to 9, **characterized in that** it has a pH of from 6.5 to 11.5.

11. Agent according to one of Claims 4 to 10, **characterized in that** it is a hair colouring agent.

## Revendications

1. Dérivés de 3-(2,5-diaminophényl)acrylamide de formule générale (I) ou sels hydrosolubles, physiologiquement acceptables, de tels dérivés 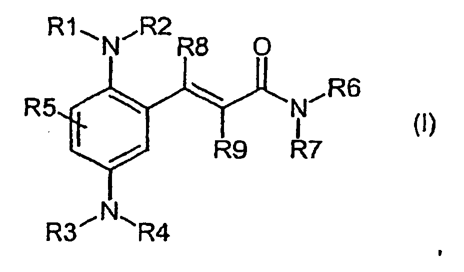 formule dans laquelle
**R1, R2, R3** et **R4** peuvent être identiques ou différents et représentent, indépendamment les uns des autres, un atome d'hydrogène, un groupe alkyle en C₁-C₆, un groupe hydroxyalkyle en C₂-C₄ ou un groupe dihydroxyalkyle en C₃-C₄, ou bien **R1** et **R2** ou, respectivement, **R3** et **R4,** forment un cycle aliphatique à 4 à 8 chaînons, au moins deux des radicaux **R1** à **R4** représentant un atome d'hydrogène ;
**R5** représente un atome d'hydrogène ou d'halogène, un groupe alkyle en C₁-C₄, un groupe hydroxyalkyle en C₁-C₄ ou un groupe alcoxy en C₁-C₄ ;
**R6** et **R7** peuvent être identiques ou différents et représentent, indépendamment l'un de l'autre, un atome d'hydrogène, un groupe alcoxy en C₁-C₂, un groupe alkyle en C₁-C₆, un groupe alkyle en C₃-C₆ insaturé, un groupe hydroxyalkyle en C₂-C₄, un groupe dihydroxyalkyle en C₃-C₄, un groupe aminoalkyle en C₂-C₄, un groupe diméthylaminoalkyle(C₂-C₄), un groupe acétylaminoalkyle(C₂-C₄), un groupe méthoxyalkyle(C₂-C₄), un groupe éthoxy-alkyle(C₂-C₄), un groupe cyanoalkyle(C₁-C₄), un groupe carboxyalkyle(C₁-C₄), un groupe aminocarbonylalkyle(C₂-C₄), un groupe pyridylméthyle, un groupe furfuryle, un groupe furfuryle hydrogéné, un groupe pyridyle substitué, un radical de formule (II) 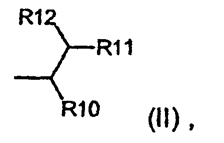 un radical de formule (III) 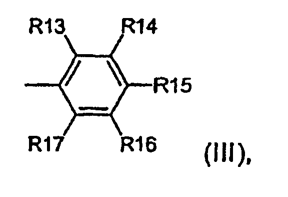 un radical de formule (IV) 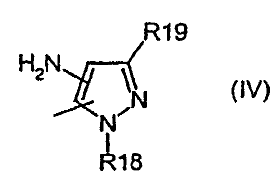 ou **R6** et **R7** forment un cycle de formule 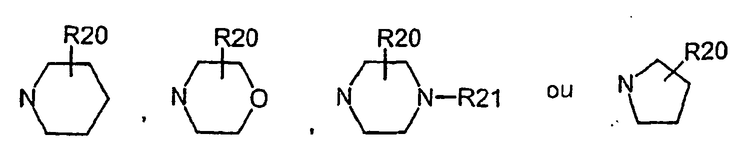 **R8, R9** et **R21** peuvent être identiques ou différents et représentent, indépendamment les uns des autres, un atome d'hydrogène ou un groupe alkyle en C₁-C₆ ;
**R10** représente un atome d'hydrogène, un groupe carboxy ou un groupe aminocarbonyle ;
**R11** et **R12** peuvent être identiques ou différents et représentent indépendamment l'un de l'autre un atome d'hydrogène, un groupe hydroxy, un groupe aminocarbonyle, un groupe méthylthiométhyle, un radical phényle substitué par un groupe phényle ou par un groupe hydroxy, ou un radical de formule 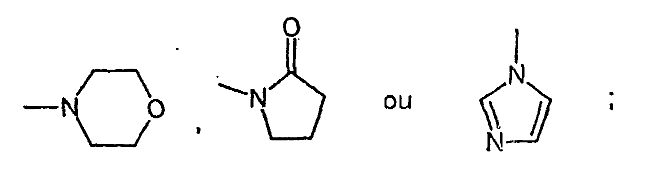 **R13, R14, R15, R16** et **R17** peuvent être identiques ou différents et représentent, indépendamment les uns des autres, un atome d'hydrogène, un atome d'halogène, un groupe cyano, un groupe hydroxy, un groupe alcoxy en C₁-C₄, un groupe hydroxyalcoxy en C₁-C₄, un groupe alkyle en C₁-C₆, un groupe alkyl(C₁-C₄)thioéther, un groupe mercapto, un groupe nitro, un groupe amino, un groupe alkylamino, un groupe hydroxyalkylamino, un groupe dialkylamino, un groupe di(hydroxyalkyl)amino, un groupe (dihydroxyalkyl)amino, un groupe (hydroxyalkyl)alkylamino, un groupe trifluorométhane, un groupe -C-(O)H, un groupe -C(O)CH₃, un groupe -C(O)CF₃, un groupe -Si(CH₃)₃, un groupe hydroxyalkyle en C₁-C₄, un groupe dihydroxyalkyle en C₂-C₄, ou deux radicaux **R13** à **R17** contigus forment ensemble un pont -O-CH₂-O- ;
**R18** représente un groupe alkyle en C₁-C₄, un groupe benzyle ou un groupe hydroxyalkyle en C₁-C₄ ;
**R19** représente un atome d'hydrogène ou un groupe alkyle en C₁-C₆ ;
**R20** représente un atome d'hydrogène, un groupe hydroxy, un groupe carboxy, un groupe aminocarbonyle ou un groupe hydroxyméthyle.

2. Dérivé de 3-(2,5-diaminophényl)acrylamide selon la revendication 1, **caractérisé en ce que** dans la formule (I) (i) un ou plusieurs des radicaux **R5**, **R8** et **R9** représente(nt) un atome d'hydrogène et/ou (ii) **R1, R2, R3** et **R4** représentent des atomes d'hydrogène et/ou (iii) **R6** représente un groupe méthyle, un groupe méthoxy ou un groupe hydroxyalkyle en C₂-C₄ et **R7** représente un groupe hydroxyalkyle en C₂-C₄ et/ou (iv) **R6** représente un atome d'hydrogène et **R7** représente un groupe alkyle en C₁-C₆, un groupe alkyle en C₃-C₆ insaturé, un groupe hydroxyalkyle en C₂-C₄, un radical furfuryle, un radical phényle substitué ou un radical pyrazole substitué, et/ou (v) **R6** et **R7** forment un cycle aliphatique de formule

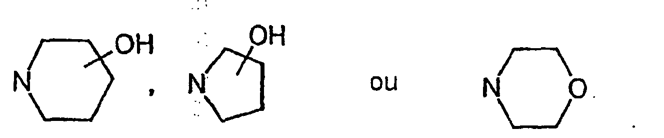

3. Dérivé de 3-(2,5-diaminophényl)acrylamide selon la revendication 1 ou 2, **caractérisé en ce qu'**il est choisi parmi le 3-(2,5-diaminophényl)-N-propylacrylamide, le 3-(2,5-diaminophényl)-N-furann-2-ylméthylacrylamide, le 3-(2,5-diaminophényl)-N-méthoxy-N-méthyl-acrylamide, le 3-(2,5-diaminophényl)-N-(2-hydroxy-1-méthyléthyl)-acrylamide, le N-cyclopropyl-3-(2,5-diaminophényl)-acrylamide, le 3-(2,5-diaminophényl)-N-isopropyl-acrylamide, le N-(4-aminophényl)-3-(2,5-diaminophényl)-acrylamide, la 3-(2,5-diaminophényl)-1-pyrrolidin-1-yl-propénone, le N-[4-amino-2-(2-hydroxyéthyl)-2H-pyrazol-3-yl]-3-(2,5-diaminophényl)-acrylamide, le N-(4-amino-2(3)-méthylphényl)-3-(2,5-diaminophényl)-acrylamide, le N-{4-[bis(2-hydroxyéthyl)-amino]phényl}-3-(2,5-diaminophényl)-acrylamide, le 3-(2,5-diaminophényl)-N-(4-hydroxyphényl)-acrylamide, la 3-(2,5-diaminophényl)-1-(3-hydroxypyrrolidin-1-yl)-propénone, le N-[5-amino-2(4)-(2-hydroxyéthoxy)-phényl]-3-(2,5-diaminophényl)-acrylamide et le 3-(2,5-diaminophényl)-N-éthylacrylamide.

4. Composition pour la teinture par oxydation de fibres de kératine, à base d'une association coupleur-développeur, **caractérisée en ce qu'**elle contient en tant que développeur au moins un dérivé de 3-(2,5-diaminophényl)acrylamide de formule (I) selon l'une quelconque des revendications 1 à 3.

5. Composition selon la revendication 4, **caractérisée en ce qu'**elle contient le dérivé de 3-(2,5-diaminophényl)acrylamide de formule (I) en une quantité de 0,005 à 20,0 % en poids.

6. Composition selon la revendication 4 ou 5, **caractérisée en ce que** le coupleur est choisi dans le groupe constitué par la N-(3-diméthylaminophényl)urée, la 2,6-diaminopyridine, le 2-amino-4-[(2-hydroxyéthyl)amino)anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)-pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)-benzène, le 1,3-diamino-4-(2,3-dihydroxy-propoxy)-benzène, le 2,4-diamino-1,5-di(2-hydroxyéthoxy)-benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]aniline, la 3-[(2-aminoéthyl)-amino]aniline, le 2,3-di(2,4-diaminophénoxy)-propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)-amino]-4-méthoxy-2-méthylphénol, le 5-[(2-hydroxyéthyl)amino]-2-méthylphénol, le 3-[(2-hydroxyéthyl)amino]phénol, le 3-[(2-méthoxyéthyl)amino)phénol, le 5-amino-2-éthylphénol, le 5-amino-2-méthoxyphénol, le 2-(4-amino-2-hydroxyphénoxy)éthanol, le 5-[(3-hydroxypropyl)-amino)-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphtol, le 2-méthyl-1-naphtol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione, N-(3-diméthylamino-phényl)urée, la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)-pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)-benzène, le 1,3-diamino-4-(2,3-dihydroxy-propoxy)-benzène, le 1,3-diamino-4-(3-hydroxypropoxy)benzène, le 1,3-diamino-4-(2-méthoxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]aniline, la 3-[(2-aminoéthyl)-amino]aniline, le 1,3-di(2,4-diaminophénoxy)-propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)-amino]-4-méthoxy-2-méthylphénol, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 5-amino-2-méthoxyphénol, le 2-(4-amino-2-hydroxyphénoxy)éthanol, le 5-[(3-hydroxypropyl)-amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, la 2,6-dihydroxy-3,4-diméthylpyridinele 5-amino-4-chloro-2-méthylphénol, le 1-naphtol, le 2-méthyl-1-naphtol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

7. Composition selon l'une quelconque des revendications 4 à 6, **caractérisée en ce qu'**en plus du dérivé de 3-(2,5-diaminophényl)acrylamide de formule (I), elle contient au moins un autre développeur choisi dans le groupe constitué par le 1,4-diaminobenzène, le 2,5-diaminotoluène, le 1-(2,5-diaminophényl)éthanol, le 2-(2,5-diaminophényl)éthanol, le 4-aminophénol et ses dérivés, les dérivés de 4,5-diaminopyrazole et les tétraaminopyrimidines.

8. Composition selon l'une quelconque des revendications 4 à 7, **caractérisée en ce que** les révélateurs et les coupleurs sont contenus chacun, par rapport à la quantité totale de la composition de teinture par oxydation, en une quantité totale de 0,005 à 20 % en poids.

9. Composition selon l'une quelconque des revendications 4 à 8, **caractérisée en ce qu'**en outre elle contient au moins un colorant direct.

10. Composition selon l'une quelconque des revendications 4 à 9, **caractérisée en ce qu'**elle présente un pH de 6,5 à 11,5.

11. Composition selon l'une quelconque des revendications 4 à 10, **caractérisée en ce qu'**il s'agit d'une composition de teinture pour cheveux.
